# EUROPEAN PATENT APPLICATION

(11) **EP 1 122 315 A1**
(43) Date of publication of application: **08.08.2001**
(21) Application number: 01101940.3
(22) Date of filing: 29.01.2001
(51) Int. Cl.: C12N 15/53, C12N 9/02, C12P 7/26

(54) **Corynebacterium aquaticum levodione reductase gene**

(30) Priority: 01.02.2000 EP 00101665
(71) Applicant: F. HOFFMANN-LA ROCHE AG, 4070 Basel (CH)
(72) Inventor: Shimizu, Sakayu, Ukyo-Ku, Kyoto-shi, Kyoto-fu (JP); Wada, Masaru, San Diego, CA 92121 (US)
(74) Representative: Müller, Ingrid, Dr.

(57) **Abstract**

The present invention is directed to genetic material useful for the preparation of actinol such as an isolated DNA comprising a nucleotide sequence coding for an enzyme having levodione reductase activity, a polypeptide encoded by such a DNA, recombinant orgainsms and the like. Those novel genetic materials may be orginated from Corynebacterium, Cellulomonas, Planococcus, Arthrobacter and the like. The present invention also provides a process for the production of actinol.

## Description

### FIELD OF THE INVENTION

The present invention relates to a DNA encoding for a novel enzyme, namely levodione reductase, an expression vector comprising said DNA, a recombinant vector comprising said DNA, a microorganism into which said DNA has been introduced, and a method for producing (4R, 6R)-4-hydroxy-2,2,6-trimethylcyclohexanone (hereinafter referred to as actinol) from (6R)-2,2,6-trimethyl-1,4-cyclohexanedione (hereinafter referred to as levodione) using said microorganism. Actinol is a useful chiral building block of naturally occurring optically active compounds such as zeaxanthin.

### BACKGROUND OF THE INVENTION

European Patent Application No. 98115564.1, filed on August 19, 1998, discloses a process for the manufacture of actinol which comprises contacting levodione with a microorganism which is selected from the group consisting of microorganisms of the genera Cellulomonas, Corynebacterium, Planococcus and Arthrobacter and which is capable of selective asymmetric reduction of levodione to actinol, and recovering the resulting actinol from the reaction mixture. It turned out that in this process one of the best strains was Corynebacterium aquaticum AKU611 (FERM BP-6448) which strain has been deposited with the National Institute of Bioscience and Human-Technology, Agency of Industrial Science and Technology, Japan in the name of F. Hoffmann-La Roche AG of Grenzacherstrasse 124, CH-4070 Basel, Switzerland on August 4, 1998 under the Budapest Treaty.

European Patent Application No. 99102037.1, filed on February 1, 1999 describes a novel enzyme, levodione reductase, that acts on levodione to produce actinol, which enzyme was isolated from Corynebacterium aquaticum AKU611 (FERM BP-6448). This enzyme is characterized by the following physico-chemical properties: 1) The levodione reductase catalyzes regio- and stereoselective reduction of levodione to actinol. 2) The relative molecular mass of the enzyme is estimated to be 142,000-155,000 ± 10,000 Da consisting of four homologous subunits having a molecular mass of 36,000 ± 5,000 Da. 3) The optimum temperature is 15-20 °C at pH 7.0 and the optimum pH is 7.5. 4) The enzyme requires NAD⁺ or NADH as a cofactor and is highly activated by monovalent cations, such as K⁺, Na⁺, Cs⁺, Rb⁺ and NH₄⁺

### SUMMARY OF THE INVENTION

The present invention relates to a DNA sequence encoding for a novel enzyme, levodione reductase, which is be useful for the preparation of actinol, an important intermediate for the production of zeaxanthin.

The isolated DNA sequence may be more specifically characterized in that
(a) the said nucleotide sequence codes for said enzyme having the amino acid sequence shown in SEQ ID No.: 1, or
(b) the said nucleotide sequence codes for a variant of the said enzyme selected from (i) an allelic variant or (ii) an enzyme having one or more amino acid addition, insertion, deletion and/or substitution but having still the same type of enzymatic activity. Particularly specified isolated DNA sequence mentioned above may be that which can be derived from a gene of Corynebacterium aquaticum and is selected from
   (i) a nucleotide sequence represented in SEQ ID No. : 2 ;
   (ii) a nucleotide sequence which, because of the degeneracy of the
   (iii) genetic code, encodes a levodione reductase having the same amino acid sequence as that encoded by the nucleotide sequence (i); and
   (iv) a nucleotide sequence which hybridizes to the complement of the nucleotide sequence from (i) or (ii) under standard hybridizing conditions.

Further provided by the present invention is a recombinant DNA which contains a sequence coding for levodione reductase and which is obtainable by the gene recombination of the isolated DNA of the present invention described above. Such a recombinant DNA may preferably be in the form of a vector. The said recombinant DNA may contain the regulatory regions such as promoter and terminator as well as open reading frame of above gene.

The present invention also provides the use of the said recombinant DNA to transform a host organism. A convenient form of the recombinant DNA may be a vector. The host organism transformed with the recombinant DNA may be useful in the improvement of the production process of actinol. Thus the present invention also provides such a recombinant organism.

The present invention further provides a method for the biological production of actinol which comprises a recombinant DNA of the present invention into an appropriate host organism and cultivating thus obtained recombinant organism in the presence of levodione as a substrate.

Moreover, the present invention provides a method for the biological production of actinol which comprises introducing a recombinant DNA described above into an appropriate host organism and cultivating thus obtained recombinant organism in the presence of levodione as a substrate. Thus the method for producing actinol being characterized in that a recombinant organism described above is cultivated under conditions conductive to the production of the actinol is one of the aspect of the present invention. This method may be applied to the biological production of actinol.

### DETAILED DESCRIPTION OF THE INVENTION

Hereinafter, the present invention is described in more detail.

### 1. Definitions

As used herein, "levodione reductase" refers to a polypeptide which is capable of catalyzing the conversion of levodione regio- and stereoselectively to actinol in the presence of NADH. European Patent Application No.99102037.1, filed on February 1, 1999, describes the physico-chemical properties of the levodione reductase. The levodione reductase of the invention can be prepared by cultivating an appropriate microorganism in an aqueous nutrient medium under aerobic conditions, disrupting the cells of the microorganism and isolating and purifying the levodione reductase from the cell-free extract of the disrupted cells of the microorganism. Many species have been found to catalyze said conversion, including the genera Cellulomonas, Corynebacterium, Planococcus and Arthrobacter. A preferred strain for the enzyme is Corynebacterium aquaticum AKU611 (FERM BP-6448).

As used herein, an "allele" or "allelic" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone, or in combination with the others, one or more times in a given sequence. A "variant" of levodione reductase, as used herein, refers to an amino acid sequence that is altered by one or more amino acids. The variant may have "conservative" changes, wherein a substituted amino acid has similar structural or chemical properties, e.g., replacement of leucine with isoleucine. More rarely, a variant may have "non-conservative" changes, e.g., replacement of a glycine with a tryptophan. Similar minor variations may also include amino acid deletions or insertions, or both. A "deletion", as used herein, refers to a change in either amino acid or nucleotide sequence in which one or more amino acid or nucleotide residues, respectively, are absent. An "insertion" or "addition", as used herein, refers to a change in an amino acid or nucleotide sequence resulting in the addition of one or more amino acid or nucleotide residues, respectively, as compared to the naturally occurring molecule. A "substitution", as used herein, refers to the replacement of one or more amino acids or nucleotides by different amino acids or nucleotides, respectively.

"Expression vector" includes vectors which are capable of expressing DNA sequences contained therein where such sequences are operably linked to other sequences capable of effecting their expression. It is implied, although not explicitly stated, that expression vectors must be replicable in the host organisms either as episomes or as an integral part of a chromosomal DNA. Clearly, a lack of replication would render them effectively inoperable. In sum, "expression vector" is also given a functional definition. Generally, expression vectors of utility in DNA recombinant techniques are often in the form of "plasmids" Plasmids refer to either circular double stranded DNA molecules or circular single stranded DNA molecules, containing an origin of replication derived from a filamentous bacteriophage. These DNA molecules, in their vector form, are not linked to the chromosomes. Other effective vectors commonly used are phage and non-circular DNA. In the present specification, "plasmid" and "vector" are often used interchangeably. However, the invention is intended to include such other forms of expression vectors which serve equivalent functions and which are, or subsequently become, known.

"Recombinant host cells", "host cell", "cells", "cell cultures" and so forth are used interchangeably to designate individual cells, cell lines, cell cultures, and harvested cells which have been or are intended to be transformed with the recombinant vectors of the invention. The terms also include the progeny of the cells originally receiving the vector.

"Transformed" or "Transformation" refers to any process for altering the DNA content of the host.

The term "standard hybridising conditions" refers to such conditions under which the person skilled in the art obtains a specific signal. The conditions may range from low stringency conditions (6x SSC, 50°C; overnight; washing in 6x SSC at room temperature) to the preferred medium stringency conditions (6x SSC; 65°C; overnight; washing in 6X SSC at 30°C to the most preferred high stringency conditions (6x SSC; 75°C; overnight; two times washing in 6x SSC at 37°C). Alternatively the degree of similarity can be determined by the percentage of homology. For the determination of homology the two sequences to be checked are aligned to each other by a suitable computer program. By using standard conditions of the program the claimed sequences have a percentage of homology of at least 40%, preferably at least 60% and more preferable at least 80% with the sequences disclosed in the present application.

The amino acids are identified by either the single-letter or three-letter designations:

| ABBREVIATIONS | |
|---|---|
| AMINO ACIDS | |
| A = Ala = Alanine | V = Val = Valine |
| L = Leu = Leucine | I = Ile = Isoleucine |
| P = Pro = Proline | F = Phe = Phenylalanine |
| W = Trp = Tryptophan | M = Met = Methionine |
| G = Gly = Glycine | S = Ser = Serine |
| T = Thr = Threonine | C = Cys = Cysteine |
| Y = Tyr = Tyrosine | N = Asn = Asparagine |
| Q = Gln = Glutamine | D = Asp = Aspartic Acid |
| E = Glu = Glutamic Acid | K = Lys = Lysine |
| R = Arg = Arginine | H = His = Histidine |

Following is a list of commercial suppliers for materials used in this invention.
a. Invitrogen: 1600 Faraday Avenue, Carlsbad, CA92008, USA
b. Amersham Pharmacia Biotech: SE-751 84 Upsara, Sweden
c. Toyobo: 2-2-8 Dojimahama, Kita-ku, Osaka, Japan
d. Takara Shuzo: 2-15-10 Nihonbashi, Chuo-ku, Tokyo, Japan
e. Promega: 2800 Woods Hollow Road, Madison, WI, USA
f. BIO101: 2251 Rutherford Rd., Carlsbad, CA92008, USA
g. PE Biosystems: 850 Lincoln Center Drive, Foster City, CA94404, USA
h. Shimadzu: 1 Nishinokyo, Kuwabaracho, Nakagyo-ku, Kyoto, Japan
i. Shinwa Chemical Industries: 50 Keishocho, Fushimi-ku, Kyoto, Japan
j. Wako Pure Chemicals: 3-1-2 Doshoumachi, Chuo-ku, Osaka, Japan
k. Oriental Yeast: 3-6-10 Shodosawa, Itabashi-ku, Tokyo, Japan
l. Amano Pharmaceuticals: 1-2-7 Nishiki, Naka-ku, Nagoya, Japan

### 2. Levodione reductase gene

The gene encoding for levodione reductase of the invention is a DNA comprising a nucleotide sequence coding for a polypeptide having an enzyme activity to convert levodione to actinol. A typical example of this gene is a levodione reductase gene which can be cloned from Corynebacterium aquaticum AKU611 (FERM BP-6448). This is a DNA comprising a nucleotide sequence coding for a polypeptide comprising the amino acid sequence as shown in SEQ ID No.: 1 of the sequence listing.

The DNA sequence may be cloned from a strain of Corynebacterium aquaticum AKU611 (FERM BP-6448), or another related organism and thus, for example, may be an allelic or species variant of the levodione reductase encoding region of the DNA sequence. Also included within the scope of the present invention is a derivative of a DNA sequence with addition, insertion, deletion and/or substitution of different nucleotides resulting in a polypeptide that encodes the same or a functionally equivalent levodione reductase. The encoded protein may also contain addition, deletion, insertion and/or substitution of amino acid residues which produce a silent change and result in a functionally equivalent levodione reductase.

The levodione reductase gene product, i.e. levodione reductase of the present invention has, as described above, an enzyme activity to convert levodione to actinol. The gene for such an enzyme activity has not been known. On the other hand, by using the levodione reductase gene, it is possible to render a microorganism such as E.coli an ability to convert levodione to actinol.

### 3. Acquisition of the DNA

The present invention provides an isolated DNA sequence which codes for an enzyme, levodione reductase, which is involved in the conversion of levodione to actinol in a microorganism. The said DNA of the present invention can mean a genomic DNA which contains regulatory sequences such as its promoter and terminator which are involved in the expression of the gene of interest, and also a cDNA which contains only open reading frame flanked between the short fragments in its 5'- and 3'- untranslated region.

The levodione reductase gene, the recombinant expression vector, and the recombinant organisms utilized in the present invention may be obtained by the following steps:
(1) Isolating chromosomal DNA from a microorganism which can provide levodione reductase of the present invention and constructing the gene library with the chromosomal DNA.
(2) Cloning the levodione reductase gene from a chromosomal DNA by colony- or plaque-hybridization, PCR cloning, Southern-blot hybridization and the like.
(3) Determining nucleotide sequence of the levodione reductase gene obtained as above by usual methods and constructing recombinant expression vectors which contain and express the levodione reductase gene efficiently.
(4) Constructing recombinant organisms carrying the levodione reductase gene on recombinant expression vectors or on chromosomes by transformation, transduction, transconjugation and electroporation.

The techniques used to isolate or clone a DNA encoding levodione reductase of the present invention are known in the art and include isolation from genomic DNA. The cloning of the DNA sequence of the present invention from such genomic DNA can be effected by using the degenerate polymerase chain reaction (hereinafter referred to as PCR).

In order to clone the levodione reductase gene, knowledge of the amino acid sequence of levodione reductase may be necessary. To accomplish this, levodione reductase protein may be purified and partial amino acid sequence may be determined by conventional method (Biosci. Biotechnol. Biochem. 62, 280-285, 1998). Determination of the complete amino acid sequence is not necessary. Once suitable amino acid sequences have been identified, oligonucleotides as primers for PCR are synthesized on the basis of information on said partial amino acid sequence. In this invention, the primers used for cloning of levodione reductase gene by PCR are based on the amino acid sequence of the internal peptide fragments of the purified levodione reductase from the genera including, but not to limited to, Corynebacterium, Cellulomonas, Planococcus and Arthrobacter, and in the most preferred embodiment, from Corynebacterium aquaticum AKU611 (FERM BP-6448). A DNA fragment (a partial DNA sequence) for levodione reductase is generated by PCR amplification with said primers and the template of Corynebacterium aquaticum chromosomal DNA. The amplified DNA fragment can be used as the probe to clone a genomic fragment coding for the whole Corynebacterium aquaticum AKU611 (FERM BP-6448) levodione reductase.

An entire gene containing its coding region as well as its regulation region such as a promoter or terminator can be cloned from a chromosome by screening of genomic library which is constructed in phage vector or plasmid vector in an appropriate host, by using a partial DNA fragment obtained by PCR as described above as a probe after it was labeled. Generally, E. coli as a host strain and E. coli vector, a phage vector such as λ phage vector, a plasmid vector, or a yeast vector is often used in the construction of library and a following genetic manipulation such as a sequencing, a restriction digestion, a ligation and the like. Identification of the desired clones from the plasmid or phage library is the best effected by a probe such that the desired gene will hybridize to a probe having a portion of the desired gene under suitable stringency conditions. In this invention, a genomic library of Corynebacterium aquaticum AKU611 (FERM BP-6448) was constructed in pYES2. In this invention, PCR-amplified fragment which was used for a probe was labeled with horseradish peroxidase (hereinafter referred to as HRP) instead of conventional ³²P label, following the protocol which was prepared by the supplier (Amersham Pharmacia Biotech). A genomic library constructed from the chromosome of Corynebacterium aquaticum AKU611 (FERM BP-6448) was screened by using an HRP-labeled DNA fragment which had a portion of a gene of interest as a probe. Hybridized colonies were picked up and used for further study. After the isolation of positive colonies, insert fragment was subcloned into an appropriate plasmid vector which can be conveniently used for sequencing. In this invention, the insert fragment in the positive vector was subcloned into pUC18 vector.

Nucleotide sequence of the object gene can be determined by a well-known method such as dideoxy chain-termination method (Proc. Natl. Acad. Sci. USA, 74, 5463-5467, 1977).

The isolated DNA sequence of the present invention may be used to identify and clone DNA encoding polypeptide having levodione reductase activity from other strains of different genera or species according to methods well known in the art.

### 4. DNA construct and transformation of a microorganism

The present invention also relates to a recombinant DNA, preferably a vector and/or plasmid comprising a sequence coding for levodione reductase. The said recombinant DNA vector and/or plasmid may comprise the regulatory regions such as promoters and terminators as well as open reading frames of above mentioned DNA.

Methods which are well known to those skilled in the art may be used to construct expression vectors containing sequence encoding levodione reductase and appropriate transcriptional and translational regulatory elements including all components which are necessary or advantageous for expression of the coding sequence of the nucleotide sequence as described in Ausubel F.M. et al.(1989), Current Protocols in Molecular Biology, John Wiley & Sons, New York, N.Y. Specific initiation and termination signals may also be used to achieve more efficient translation of sequences encoding levodione reductase.

An isolated DNA sequence encoding levodione reductase may be manipulated in a variety of ways to provide for expression of the polypeptide. Manipulation of the nucleotide sequence encoding said levodione reductase prior to its insertion into a vector may be desirable or necessary depending on the expression vector. The techniques for modifying nucleotide sequences utilizing cloning methods are well known in the art.

A variety of expression vector/host systems may be utilized to contain and express sequences encoding levodione reductase. These include, but are not limited to, microorganisms such as bacteria transformed with recombinant bacteriophage, plasmid, or cosmid DNA expression vectors; yeast transformed with yeast expression vectors; plant cell systems transformed with virus expression vectors or with bacterial expression vectors; or animal cells. An expression vector may be selected depending upon the use intended for the levodione reductase. For example, when large quantities of levodione reductase are needed, vectors which direct high level expression of the introduced DNA sequence may be used. Such vectors include, but are not limited to, the E. coli cloning and expression vectors such as pBluescript II and pUC18.

The host cell which is transformed with the DNA sequence coding for levodione reductase of the invention may be either eukaryotic cells or prokaryotic cells. The choice of a host cell may to a large extent depend on the gene encoding the polypeptide and its source. Suitable prokaryotic host cells are in particular bacterial cells such as E. coli which are used to provide for high level expression of the protein. In order to overexpress an enzyme of interest, appropriate promoter systems contemplated for use in high level expression may be used. Such promoters include, but are not limited to, the lac or T7 expression system.

### 5. Production of actinol by a recombinant organism

The present invention also provides the use of the said recombinant DNA, vector or plasmid, to transfer a host organism. The recombinant organism obtained by use of the recombinant DNA is capable of overexpressing DNA sequence encoding levodione reductase. The host organism transformed with the recombinant DNA may be useful in the production process of actinol. Thus the present invention also provides such a recombinant organism/transformed host.

### 6. Production of levodione reductase by a recombinant organism

The present invention also relates to a method for producing the levodione reductase, which comprises culturing a recombinant organism mentioned above, under a condition conductive to the production of said enzyme and relates also to the enzyme itself. Host cells transformed with nucleotide sequences encoding levodione reductase may be cultured under conditions suitable for the expression and recovery of the protein from cell culture.

The recombinant organism may be cultured in a nutrient medium containing saccharides such as glucose and sucrose, alcohols such as ethanol and glycerol, fatty acids such as oleic acid and stearic acid, or esters thereof, or oils such as rapeseed oil and soybean ail as carbon sources ; ammonium sulfate, sodium nitrate, peptone, amino acids, corn steep liquor, bran, yeast extract and the like as nitrogen sources ; magnesium sulfate, sodium chloride, calcium carbonate, potassium monohydrogen phosphate, potassium dihydrogen phosphate and the like as inorganic salt sources ; and malt extract, meat extract and the like as other nutrient sources. The cultivation can be carried out aerobically, aerobically, normally for a period of 1 to 7 days at a medium pH of 3 to 9 and a cultivation temperature of 10 to 40°C.

The levodione reductase produced by a recombinant cell may be secreted or contained intracellularly depending on the sequence and/or the vector used. The levodione reductase may then be isolated from culture medium or a host cell by conventional procedures.

An embodiment for isolation and purification of levodione reductase from the recombinant cells after the cultivation is as follows :
(1) Cells are harvested from the liquid culture broth by centrifugation or filtration.
(2) The harvested cells are washed with water, physiological saline or a buffer having an appropriate pH.
(3) The washed cells are suspended in the buffer solution and disrupted by means of a homogenizer, sonicator, French press or treatment with lysozyme and the like to give a solution of disrupted cells.
(4) The levodione reductase is isolated and purified from the cell-free extract of disrupted cells.

Following confirmation of the enzyme activity, the expressed levodione reductase protein would be used for raising of the antibody to the purified enzyme. Antibody thus prepared would be used for a characterization of the expression of the corresponding enzyme in a strain improvement study, an optimization study of the culture condition, and the like.

### Examples

The following materials and methods were employed in the Example described below:

### Strains

Corynebacterium aquaticum AKU611 (FERM BP-6448)

E. coli DH5α: [φ80δlacZΔM15, F⁻, λ⁻, Δ(lacZYA-argFVl69), hsd R17(r_{K}⁻, m_{K}⁺), recAl, endAl, supE44, deoR, thi-1, gyrA96, relAl] (Toyobo, Osaka, Japan). E. coli JM109: [recAl, endAl, gyrA96, thi, hsdR17(r_{K}⁻, m_{K}⁺), mcrB⁺, supE44, relAl, Δ(lac-proAB), F'(traD36, proAB, lacI^{q}, lacZΔ M15), λ⁻ ] (Toyobo, Osaka, Japan).

### Vectors

pYES2 (Invitrogen)
pGEM-T (Promega)
pUC18 (Toyobo)

### Primers

| | |
|---|---|
| T-7 primer | 5'-TAATACGACTCACTATAGGG-3' |
| SP6 primer | 5'-TACGATTTAGGTGACACTAT-3' |
| M13 primer M4 | 5'-GTTTTCCCAGTCACGAC-3' |
| M13 primer RV | 5'-CAGGAAACAGCTATGAC-3' |

### Media

Corynebacterium aquaticum AKU611 (FERM BP-6448) was cultured aerobically at 30 °C for 20 h in a medium (pH 7.0) containing 1% glucose, 1.5% peptone, 0.3% K₂HPO₄,0.1% yeast extract, 0.2% NaCl, and 0.02% MgSO₄ 7H₂O. E. coli transformants containing the Corynebacterium aquaticum AKU611 (FERM BP-6448) levodione reductase gene were grown in Luria-Bertain medium (LB medium) consisting of 10 g of tryptone, 10 g of sodium chloride, and 5 g of yeast extract (pH 7.2)/liter, or in M9 medium (page A-3, Molecular Cloning, 1989, Cold Spring Harbor Laboratory Press) supplemented with casamino acids.

### Enzymes and Chemicals

Restriction endonucleases and other DNA-modifying enzymes were obtained from Takara Shuzo and Toyobo. Ex taq, a Taq DNA polymerase and Ex taq buffer were purchased from Takara Shuzo. ECL direct nucleic acid labelling and detection system were purchased from Amersham Pharmacia Biotech.

### Methods

General methods of molecular genetics were practiced according to Molecular cloning: a Laboratory Manual, 2nd Edition (Cold Spring Harbor Laboratory Press, 1989).

Isolation of a chromosomal DNA from Corynebacterium aquaticum AKU611 (FERM BP-6448) was prepared using Genome Isolation Kit (BIO101).

Polymerase chain reaction (PCR) was performed with a thermal cycler (PE Biosystems). Degenerate PCR primers were synthesized by the phosphoramidite method using an Applied Biosystems Model 381A automatic synthesizer (PE Biosystems).

Nucleotide sequence analysis was performed on dideoxy chain-termination method (Proc. Natl. Acad. Sci. USA, 74, 5463-5467, 1977). A Taq dye primer sequencing kit was used with an autosequencer (DNA Sequencer 377A, PE Biosystems).

Levodione reductase activity was determined by spectrophotometrically measuring the levodione-dependent decrease of the absorbance of NADH content at 340 nm. The standard 2.5 ml assay mixture contained 5 µmole of levodione (final concentration, 2.0 mM), 0.8 µmole of NADH, 500 µmole of potassium phosphate buffer (pH 7.0), and the enzyme. One unit of the enzyme activity was defined as the amount of enzyme that catalyzed oxidation of 1 µmole of NADH per min.

Quantitative analysis of the levodione and actinol contents was performed with a Shimadzu model GC-14B GC equipped with a flame ionization detector by using a type HR-20M capillary column (0.25 mm by 30 m; Shinwa Chemical Industries) at 160 °C (isothermal) and He as the carrier gas at a flow rate of 1 ml/min. Under these conditions, levodione, actinol, and (4S,6R)-hydroxy-2,2,6-trimethylcyclohexanone (a diastereomer of actinol) eluted at 6.8, 15.6, and 15.9 min, respectively.

Purification of the levodione reductase was performed according to the procedure described in European Patent Application No. 99102037.1 filed on February 1, 1999. The purified enzyme was digested with lysyl endopeptidase (Wako Pure Chemicals) under the conditions described in Appl. Environ. Microbiol. 62, 2303-2310, 1996. The peptides were separated by reverse-phase high-performance liquid chromatography on a µRPC C2/C18 column (Amersham Pharmacia Biotech) connected to a Smart system (microscale protein purification system; Amersham Pharmacia Biotech). The peptides were eluted with a linear 0 to 80% acetonitrile gradient containing 0.1% trifluoroacetic acid.

A partial amino acid sequence was determined by automated Edman degradation with a model 476A pulsed liquid protein sequencer (PE Biosystems) as described previously (Biosci. Biotechnol. Biochem. 62, 280-285, 1998). The partial amino acid sequence obtained was compared with the sequences of proteins stored in the SWISS-PROT (release 37.0+/06- 14, June 99), PIR (release 60.0, March 99), and PRF (release 99-05, May 99) protein databases. Sequence alignment was performed by using in Blast (J. Mol. Biol. 215, 403-410, 1990) and Fasta (Proc. Natl. Acad. Sci. USA 85, 2444-2448, 1988) programs.

The following examples are provided to further illustrate the invention. These examples are illustrative only and are not intended to limit the scope of the invention in any way.

### Example 1

### Partial amino acid sequence from Corynebacterium aquaticum AKU611 (FERM BP-6448)

The N-terminal amino acid sequence of purified Corynebacterium aquaticum levodione reductase was determined by automated Edman degradation by using a model 476A pulsed liquid protein sequencer (PE Biosystems). This sequence was similar to the NH₂-terminal amino acid sequences of other short-chain dehydrogenase/reductase (SDR) family enzymes, such as biphenyl-2,3-dihydro-2,3-diol dehydrogenase of Pseudomonas sp. strain KKS102 (Biochem. Biophys. Res. Commun. 202, 850-856, 1994). Moreover, this NH₂-terminal amino acid sequence contains G-X-X-X-G-X-G, which is highly conserved in the NH₂-terminal regions of SDR family proteins. The following amino acid sequences were obtained from the N-terminal of levodione reductase:

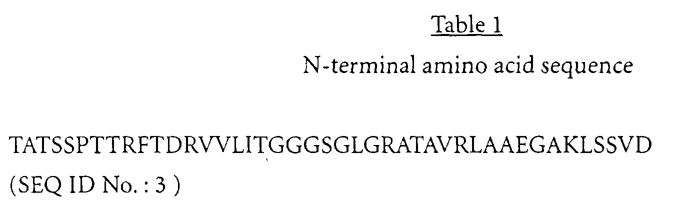

The levodione reductase protein was digested with lysyl endopeptidase, and the resulting digest was separated by the Smart system (Amersham Pharmacia Biotech). The peptides (K-1, K-2, and K-3) were isolated, and the amino acid sequences of these peptides were analyzed with a protein sequencer. The K-1, K-2, and K-3 sequences are shown in Table 2. When these sequences were compared with the sequences in three protein sequence databases (PIR, PRF, and SWISS-PROT) by using the sequence similarity search programs Blast and Fasta, the K-1 and K-2 sequences were significantly similar to partial amino acid sequences of 2,5-dichloro-2,5-cyclohexadiene-1,4-diol dehydrogenase of S. paucimobilis (J. Bacteriol. 176, 3117-3125, 1994) and 3-oxoacyl-[acyl-carrier protein] reductase of Haemophilus influenzae (Whole-genome random sequencing and assembly of Haemophilus influenzae Rd, Science 269, 495-512, 1995), respectively, both of which belongs to the SDR family. However, K-3 did not exhibit significant similarity to any other SDR family protein.

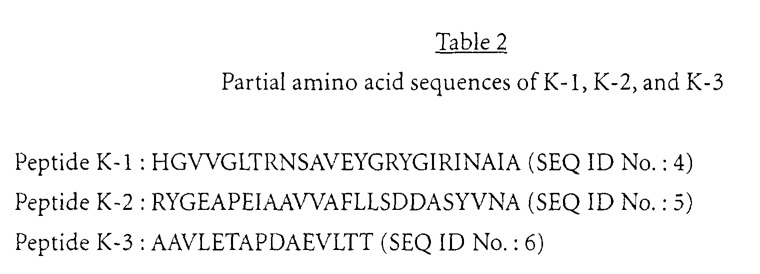

### Example 2

### Construction of a genomic DNA library

Chromosomal DNA from Corynebacterium aquaticum AKU611 (FERM BP-6448) was prepared by using Genome Isolation Kit (BIO101). Chromosomal DNA from Corynebacterium aquaticum AKU611 (FERM BP-6448) was partially digested with Sau3AI. The digested chromosomal DNA was fractionated by agarose gel electrophoresis. DNA fragment (500 ng) predominantly 2-3 kb was mixed with BamHI-digested pYES2 vector (200 ng) and ligated using Ligation high (Toyobo) at 16 °C for 30 min in vitro, and the ligation mixture was used to transform competent high E. coli DH5α cells (Toyobo). Competent E. coli DH5α cells were plated and cultured on LB agar plate containing ampicillin at 37°C overnight. The library (2,242 colonies) was obtained.

### Example 3

### Cloning of levodione reductase genomic DNA by PCR amplification

### Amplification of partial levodione reductase gene of Corynebacterium aquaticum AKU611 (FERM BP-6448)

Degenerate oligonucleotide primers were designed and synthesized based on the amino acid sequences of the internal peptide fragments (Table 2) of the levodione reductase peptide from Corynebacterium aquaticum AKU611 (FERM BP-6448). The primers, 2-23(+) and 2-23(-) were synthesized on the basis of the amino acid sequence, YGEAPEI of peptide K-2 and the primers, 1-19(+) and 1-19(-) on the basis of the amino acid sequence, AVEYGRY of peptide K-1 as shown in Table 3. The primers, 1-19(+) and 119(-) contained one inosine moiety.

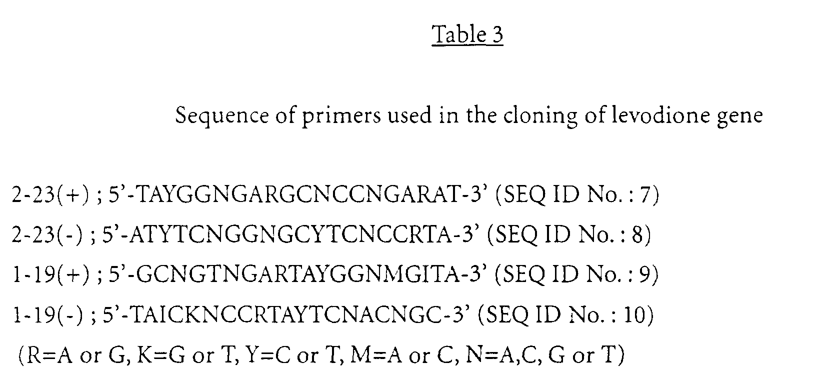

Genomic DNA was amplified by PCR using a thermal cycler (PE Biosystems). The PCR reactions (20 µl) were carried out using 100 ng of chromosomal DNA of Corynebacterium aquaticum AKU611 (FERM BP-6448) as the template, 5 µM of each degenerate primer, 312 µM each of dATP, dCTP, dGTP, and dTTP, 2.5 U (final concentration) of Ex Taq (Takara Shuzo) as a DNA polymerase, and 2 µl of EX Taq buffer (Takara Shuzo). Taq polymerase (2.5 U) were added after the reaction incubated at 95 °C for 5 minutes and reaction was then cycled 25 times as follows: 95 °C for 30 seconds, 42°C for 30 seconds, and 72 °C for 2 minutes. Various combinations of primers were tested and one combination resulted in prominent band [2-23(-) and 1-19(+)]. The reaction products were isolated and purified using agarose gel electrophoresis. The gel-purified PCR product was added by adenine tail at 3'-terminal ends, and ligated into pGEM-T vector. The ligation mixture was used to transform competent high E. coli DH5α. After cultivation of the transformant, the plasmid was extracted. The nucleotide sequence was then determined by an autosequencer using primers, T-7 and SP6 which derived from T vector. After a result of sequence, it was found that the PCR product was composed of 172 bp nucleotides (Table 4) and the deduced amino acid sequence was consistent with the internal amino acid sequence of the native levodione reductase protein.

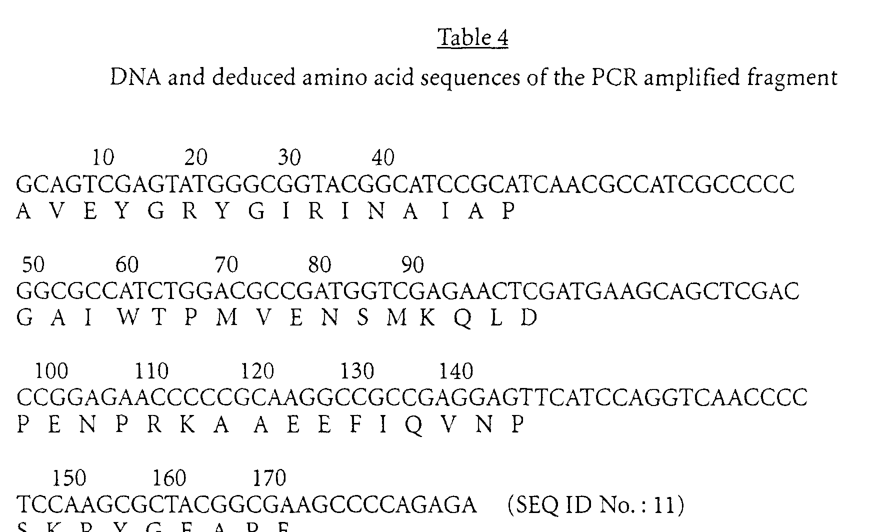

### Example 4

### Cloning of levodione reductase genomic gene from Corynebacterium aquaticum AKU611

### (1) Cloning of levodione reductase genomic gene from Corynebacterium aquaticum AKU611 (FERM BP-6448) using the PCR amplified product as a probe

The PCR amplified 172-bp fragment was used to screen a levodione reductase genomic gene from the genomic DNA library. The PCR amplified 172-bp fragment was denatured by heat treatment and labeled with horseradish peroxidase (HRP) using ECL direct nucleic acid labelling and detection system (Amersham Pharmacia Biotech) according to the manufacturer's instructions. The library (2,242 colonies) as described in Example 2 was transferred onto Hybond N+, a positively charged nylon membrane (Amersham Pharmacia Biotech) and denatured and fixed by the buffer including 0.5 M NaOH. The membranes were probed with the HRP-labeled PCR fragment. After hybridization, membranes were washed and the positive clones on membranes were detected by chemiluminescent detection system. From 2,242 colonies (recombinants) in the genomic library, twenty-five positive clones were obtained.

To investigate whether gene fragments of the levodione reductase are contained in these clones and to select the positive clone, PCR was performed with 1-19(+) and 2-23(-) as the primers, and each of twenty-five clones as a template. Reaction mixture (20 µl) contained 0.5 µM of each primer, 10 ng of a template (each of 25 clones), 312 µM of each dNTP, 2.5 U (final concentration) EX Taq (Takara Shuzo) as a DNA polymerase, and 2 µl of EX Taq buffer (Takara Shuzo). PCR conditions used are 96 °C for 1 minute followed by 25 cycles each at 95 °C for 30 seconds, 55 °C for 30 seconds, and 72 °C for 1 minute. As a result, No. 27 clone carrying an approximately 8.5 kb DNA fragment, which contained about 2.7 kb insert DNA, was selected for further analysis.

### (2) Nucleotide sequencing of No. 27 clone

DNA sequencing was performed with an autosequencer using the primer walking technique. The primers, pYES2(+) and pYES2(-) derived from pYES2, and 2-23(-), 27-2(-), and 27-1(-) derived from the internal peptide fragments were used for DNA sequencing. About 260 bp nucleotides at pYES2(+) side and about 820 bp nucleotides at pYES2(-) side were sequenced. The used primers are shown in Table 5.

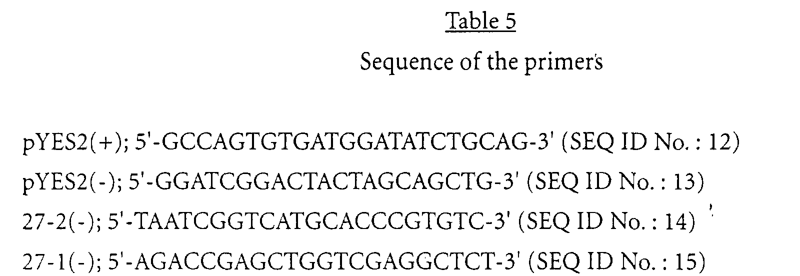

To analyze the nucleotide sequence in more detail, No 27 clone was digested with Sac I for subcloning and DNA sequencing. Two fragments with sizes of 7.5 kb and 1 kb were isolated and purified using agarose gel electrophoresis. The 7.5 kb fragment including 1.6 kb insert DNA was circularized by self-ligation using Ligation high, and the 1 kb fragment was ligated with Sac I-digested pUC18 vector using Ligation high (Toyobo). Each ligation mixture was used to transform E. coli JM109 cells. E. coli JM109 cells were placed and cultured on LB agar plate at 37 °C overnight. The nucleotide sequence of each transformant obtained was determined by an autosequencer.

### (3) Nucleotide sequencing of 1kb fragment

The nucleotide sequence was determined using M4 and RV, primers for M13 vectors, and SUB27(+), a primer derived from the sequence of this fragment. The used primers are shown in Table 6.

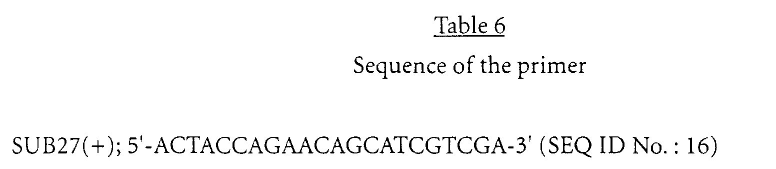

The nucleotide sequence of 1 kb fragment was consistent with that of pYES2(-) side of No. 27 clone. A portion (441 bp) of the deduced Open Reading Frame (hereinafter referred to as ORF) was present in this fragment.

### (4) Nucleotide sequence of 7.5 kb fragment

DNA sequencing of 1.6 kb insert DNA of 7.5 kb fragment was performed using pYES2(+), pYES2(-), primers for pYES2, P-2(+), P-3(+), P-4(+), SUB No.1(-), SUB No.2(-), SUB No.3(-), P-4(-), and P-3(-), primers derived from the sequences of this fragment. The used primers are shown in Table 7.

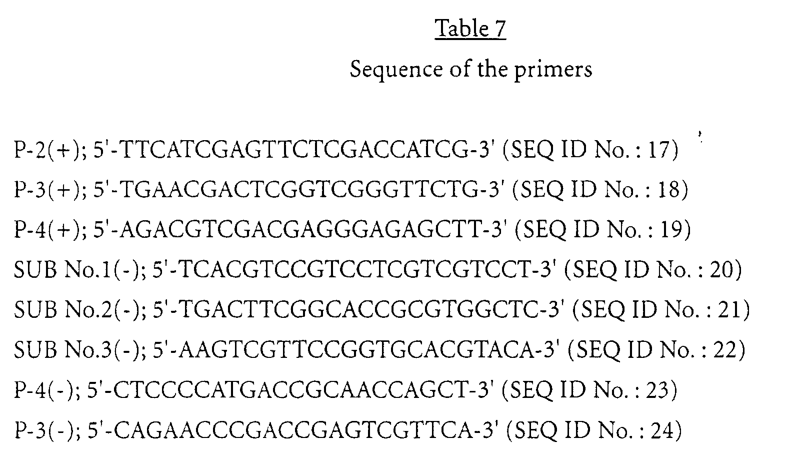

The nucleotide sequence analysis of the 1.6 kb insert DNA revealed to contain 708 bp levodione reductase gene lacking its C-terminal region. It was found that the nucleotide sequence of this fragment contained those of the internal peptide (F27), N-terminus, and first Met. The presence of SD sequence upstream of first Met was also suggested. To clone the full-length ORF, the 708 bp fragment was used to design a probe.

### (5) Construction of genomic library and screening of the full length levodione reductase gene by using 708-kb fragment as a probe

Chromosomal DNA from Corynebacterium aquaticum AKU611 (FERM BP-6448) was prepared by using Genome Isolation Kit (BIO101). Chromosomal DNA from Corynebacterium aquaticum AKU611 (FERM BP-6448) was partially digested with Sac I. The digested chromosomal DNA was fractionated by agarose gel electrophoresis. DNA fragment (500 ng) predominantly 4 kb was mixed with Sac I-digested pYES2 vector (200 ng) and ligated using Ligation high (Toyobo) at 16 °C for 30 min in vitro, and the ligation mixture was used to transform competent high E. coli DH5α cells (Toyobo). Competent E. coli DH5α cells were plated and cultured on LB agar plate containing ampicillin at 37 °C overnight. The library (974 colonies) was obtained. The 708-bp fragment obtained in Example 4 (4) was used to screen a levodione reductase genomic gene from the genomic DNA library. The 708-bp fragment was denatured by heat treatment and labeled with horseradish peroxidase (HRP) using Amersham ECL direct nucleic acid labeling and detection system (Amersham Pharmacia Biotech) according to the manufacturer's instructions. The library (974 colonies) was transferred onto Hybond N+, a positively charged nylon membrane (Amersham Japan) and denatured and fixed by the buffer including 0.5 M NaOH. The membranes were probed with the HRP-labeled PCR fragment. After hybridization, membranes were washed and the positive clone on membranes was detected by chemiluminescent detection system. From 974 colonies (recombinants) in the genomic library, fifty positive clones were obtained.

To further select the positive clone, PCR was performed with pYES2(+), pYES2(-), 2-23(-), and first Met(+) as the primers, and each of fifty clones as a template. Reaction mixture (20 µl) contained 0.5 µM of each primer, 10 ng of a template (each of 50 clones), 312 µM of each dNTP, 2.5 U (final concentration) EX Taq (Takara Shuzo) as a DNA polymerase, and 2 µl of EX Taq buffer (Takara Shuzo). PCR conditions used are 95 °C for 1 minute followed by 25 cycles each at 95 °C for 30 seconds, 55 °C for 30 seconds, and 72 °C for 1 minute. As a result, one positive clone, which contained about 3 kb insert DNA, was selected for sequencing.

DNA sequencing of a newly isolated clone was performed using P-3(-) and P-2-2(-) as primers. The sequence of the primer P-2-2(-) is shown in Table 8.

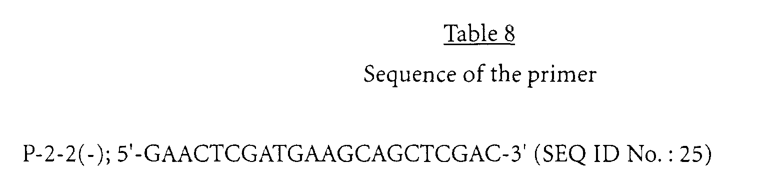

DNA sequence analysis of this clone revealed a levodione reductase gene region of 804 bp encoding a protein of 267 amino acids (SEQ ID No. : 1). The stop codon TGA was present approximately 100 bp downstream of the probe sequence. The molecular mass (27.9 KDa) of the levodione reductase was calculated by DNASIS.

The deduced amino acid sequence was compared with other protein sequences. A high level of identity was found for 2,5-DDOL-dehydrogenase (Lin C) of Sphingomonas paucimobilis UT26 (37%).

### Example 5

### Expression of levodione reductase gene and production of actinol from levodione in E. coli

### (1) Construction of a recombinant DNA harboring the levodione reductase gene

Chromosomal DNA from Corynebacterium aquaticum AKU611 (FERM BP-6448) was prepared by using Genome Isolation Kit (BIO101). On the other hand, oligonucleotide primers were designed and synthesized based on the upstream region of the N-terminal and the downstream region of the C-terminal amino acid sequences of the levodione reductase gene as shown in Table 9. EcoRI site was constructed in both primers to obtain the levodione reductase gene in an EcoRI fragment for constructing an expression vector.

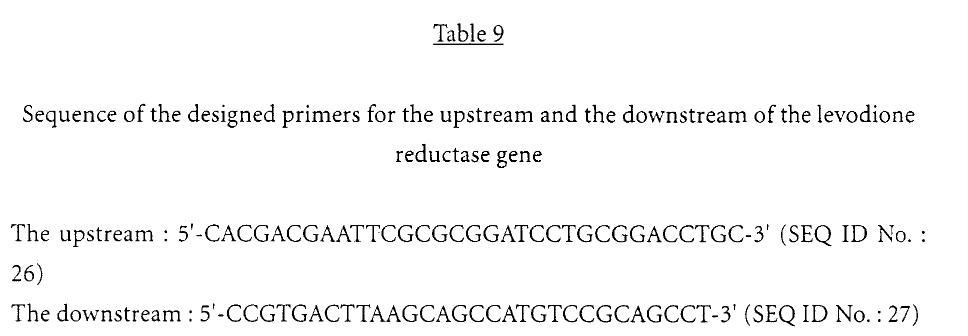

The objective DNA fragment was amplified by using a thermal cycler (PE Biosystems). The PCR reactions (20 µl) were carried out using 5 ng of a plasmid DNA including the full length levodione reductase gene as the template, 250 nmole each of the primers, 0.2 mM each of dATP, dCTP, dGTP, and dTTP, 1 U (final concentration) of Ex Taq (Takara Shuzo) as a DNA polymerase, and 2 µl of EX Taq buffer (Takara Shuzo). After the reaction mixture was incubated at 94 °C for 1 minute, the reaction was then cycled 25 times as follows: 98 °C for 20 seconds, 70°C for 2 minutes, and 72 °C for 4 minutes. The reaction product was isolated and cut by an EcoRI restriction enzyme. Then the EcoRI fragment thus obtained was ligated with EcoRI-digested pUC18 vector. The obtained recombinant DNAs designated as pUC3-6 and pUC3-5. In pUC3-6, the levodione reductase gene was inserted with opposite direction to the lac promoter of the pUC18. On the other hand, in pUC3-5, the levodione reductase gene was inserted with the same direction as that of the lac promoter of the pUC18.

### (2) Expression of the levodione reductase gene in E. coli

The recombinant DNAs pUC3-5 and pUC3-6 were used to transform E. coli JM109. The transformants thus obtained, E. coli JM109/pUC3-5 and E. coli JM109/pUC3-6, were cultivated in 3 ml of LB medium supplemented with 50 µg/ml of ampicillin. Ten µl of the above culture was inoculated into 10 ml of the same medium as above, and incubated for overnight at 37 °C. From this culture, 5 ml of the cultured broth was centrifuged to separate the cells. With these procedures, the sample cells of E. coli JM109/pUC3-5 and E. coli JM109/pUC3-6 were prepared.

A reaction mixture (1 ml),(Concentration of the components below are written as final concentration) consisting of the cells, 100 mM potassium phosphate buffer (pH 7.0), 0.6 mg/ml NAD⁺ (Oriental Yeast), 31.2 unit/ml glucose dehydrogenase (Amano Pharmaceuticals), 5% (w/v) D-glucose, and 0.5% (w/v) levodione was shaken at 30 °C for 24 hours. After the reaction, the reaction mixture was extracted with 1 ml of ethylacetate and concentrated. The extract was analyzed by gas chromatography [ column: HR-20M (Shinwa Chemical Industries) 0.25 mmφ x 30m, column temperature: 160°C (constant), injector temperature : 250°C, carrier gas: He (ca. 1ml/min)]. The yield and the optical purity of the product are summarized in Table 10.

**Table 10**

| Production of actinol from levodione | | |
|---|---|---|
| Strain | Conversion Yield (%) | Optical Purity (% e.e. for actinol) |
| E. coli JM109/pUC3-5 | 67.6 | 34.5 |
| E. coli JM109/pUC3-6 | 22.7 | 85 |

In addition, by applying the cells of E. coli JM109/pUC3-5 and E. coli JM109/pUC3-6 prepared by using a M9 medium supplemented by casamino acids, the above reaction was carried out under the same conditions above. The results are shown in Table. 11.

**Table 11**

| Production of actinol from levodione | | |
|---|---|---|
| Strain | Conversion Yield (%) | Optical Purity (% e.e. for actinol) |
| E. coli JM109/pUC3-5 | 28 | 88.1 |
| E. coli JM109/pUC3-6 | 31.7 | 87.6 |

SEQUENCE LISTING

<110> Wada, Masaru

<120> Levodione reductase gene and use thereof

<130> Levodione reductase

<140>
<141>

<160> 2

<170> PatentIn Ver. 2.1

<210> 1
<211> 267
<212> PRT
<213> Corynebacterium aquaticum

<400> 1
Met Thr Ala Thr Ser Ser Pro Thr Thr Arg Phe Thr Asp Arg Val Val
1               5                   10                  15

Leu Ile Thr Gly Gly Gly Ser Gly Leu Gly Arg Ala Thr Ala Val Arg
            20                  25                  30

Leu Ala Ala Glu Gly Ala Lys Leu Ser Leu Val Asp Val Ser Ser Glu
        35                  40                  45

Gly Leu Glu Ala Ser Lys Ala Ala Val Leu Glu Thr Ala Pro Asp Ala
    50                  55                  60

Glu Val Leu Thr Thr Val Ala Asp Val Ser Asp Glu Ala Gln Val Glu
65                  70                  75                  80

Ala Tyr Val Thr Ala Thr Thr Glu Arg Phe Gly Arg Ile Asp Gly Phe
                85                  90                  95

Phe Asn Asn Ala Gly Ile Glu Gly Lys Gln Asn Pro Thr Glu Ser Phe
            100                 105                 110

Thr Ala Ala Glu Phe Asp Lys Val Val Ser Ile Asn Leu Arg Gly Val
        115                 120                 125

Phe Leu Gly Leu Glu Lys Val Leu Lys Ile Met Arg Glu Gln Gly Ser
    130                 135                 140

Gly Met Val Val Asn Thr Ala Ser Val Gly Gly Ile Arg Gly Ile Gly
145                 150                 155                 160

Asn Gln Ser Gly Tyr Ala Ala Ala Lys His Gly Val Val Gly Leu Thr
                165                 170                 175

Arg Asn Ser Ala Val Glu Tyr Gly Arg Tyr Gly Ile Arg Ile Asn Ala
            180                 185                 190

Ile Ala Pro Gly Ala Ile Trp Thr Pro Met Val Glu Asn Ser Met Lys
        195                 200                 205

Gln Leu Asp Pro Glu Asn Pro Arg Lys Ala Ala Glu Glu Phe Ile Gln
    210                 215                 220

Val Asn Pro Ser Lys Arg Tyr Gly Glu Ala Pro Glu Ile Ala Ala Val
225                 230                 235                 240

Val Ala Phe Leu Leu Ser Asp Asp Ala Ser Tyr Val Asn Ala Thr Val
                245                 250                 255

Val Pro Ile Asp Gly Gly Gln Ser Ala Ala Tyr
            260                 265

<210> 2
<211> 804
<212> DNA
<213> Corynebacterium aquaticum

400> 2
atgaccgcaa ccagctcccc cacgacccgc ttcaccgacc gcgtcgtgct catcaccggc 60
ggcggctccg gcctcggccg tgcgaccgcc gtccgtctcg ccgccgaggg cgcgaagctc 120
tccctcgtcg acgtctcctc cgagggactc gaggcctcga aggccgccgt gctcgagacc 180
gcccccgacg ccgaggtcct caccaccgtc gccgacgtct cggacgaggc ccaggtcgag 240
gcctacgtca ccgccaccac cgagcgcttc ggccgcatcg acggcttctt caacaacgcc 300
ggcatcgagg gcaagcagaa cccgaccgag tcgttcaccg ccgccgagtt cgacaaggtc 360
gtctcgatca acctgcgcgg cgtgttcctc ggcctcgaga aggtcctgaa gatcatgcgc 420
gagcagggct ccggcatggt cgtcaacacg gcgagcgtcg gcggcatccg cggcatcggc 480
aaccagtccg gctacgccgc cgccaagcac ggggtcgtcg gtctcacccg caactccgcc 540
gtcgagtacg gccgctacgg catccgcatc aacgccatcg cccccggcgc catctggacg 600
ccgatggtcg agaactcgat gaagcagctc gacccggaga acccccgcaa ggccgccgag 660
gagttcatcc aggtcaaccc ctccaagcgc tacggcgagg cgcccgagat cgccgcggtc 720
gtcgccttcc tgctgtccga cgacgcctcg tacgtcaacg ccacggtcgt cccgatcgac 780
ggcgggcagt ccgccgcgta ctga 804

## Claims

1. An isolated DNA comprising a nucleotide sequence coding for an enzyme having levodione reductase activity.

2. The isolated DNA according to claim 1, comprising a nucleotide sequence cording for the enzyme having levodione reductase activity with the following physico-chemical properties :
1) the levodione reductase catalyzes regio- and stereoselective reduction of levodione to actinol,
2) the relative molecular mass of the enzyme is estimated to be 142,000-155,000 ± 10,000 Da consisting of four homologous subunits having a molecular mass of 36,000 ± 5,000 Da,
3) the optimum temperature is 15-20 °C at pH 7.0 and the optimum pH is 7.5,
4) the enzyme requires NAD⁺ or NADH as a cofactor and is highly activated by monovalent cations, such as K⁺, Na⁺, Cs⁺, Rb⁺ and NH₄⁺.

3. The isolated DNA according to claims 1 and 2, which is characterized in that
1) the said nucleotide sequence codes for said enzyme having the amino acid sequence shown in SEQ ID No.: 1, or
2) the said nucleotide sequence codes for a variant of the said enzyme selected from (i) an allelic variant or (ii) an enzyme having one or more amino acid addition, insertion, deletion and/or substitution but having still the same type of enzymatic activity.

4. The isolated DNA according to claims 1 and 2, which is characterized in that the said nucleotide sequence is:
1) a nucleotide sequence represented in SEQ ID No:2;
2) a nucleotide sequence which, because of the degeneracy of the genetic code, encodes a levodione reductase having the same amino acid sequence as that encoded by the nucleotide sequence (i); and
3) a nucleotide sequence which hybridizes to the complement of the nucleotide sequence from (i) or (ii) under standard hybridizing conditions.

5. A vector or a plasmid comprising a DNA as claimed in anyone of claims 1 to 4.

6. A host cell transformed or transfected by a DNA as claimed in any one of claims 1 to 4 or a vector or a plasmid as claimed in claim 5.

7. A polypeptide encoded by a DNA as claimed is anyone of claims 1 to 4.

8. A process for the production of a polypeptide as claimed in anyone of claims 1 to 4 which comprises culturing a transformed host cell as claimed in claim 6 under the conditions conductive to the production of said enzyme.

9. A process for the production of actinol which comprises contacting levodione with the polypeptide as claimed in claim 7.
